# EUROPEAN PATENT SPECIFICATION

(11) **EP 0 694 540 B1**
(45) Date of publication and mention of the grant of the patent: **19.08.1998**
(21) Application number: 95107128.1
(22) Date of filing: 11.05.1995
(51) Int. Cl.: C07D 307/52, A61K 31/34

(54) **Process for the manufacture of form 1 ranitidine hydrochloride**
Verfahren zur Herstellung von Ranitidinhydrochlorid in der Kristallform 1
Procédé pour la préparation de l'hydrochlorure de ranitidine de forme 1

(30) Priority: 24.06.1994 US 265307
(43) Date of publication of application: 31.01.1996
(73) Proprietor: Ranbaxy Laboratories Limited, New Delhi 110020 (IN)
(72) Inventor: Khanna, Jag Mohan, 74 Asian Games Village Complex, New Delhi, 110049 (IN); Kumar, Naresh, 121 South Park, New Delhi, 110019 (IN); Khera, Brij, New Delhi, 110015 (IN); Khanna, Mahavir Singh, Delhi, 110051 (IN)
(74) Representative: Cohausz & Florack

(56) References cited:
- EP-A- 0 626 381
- GB-A- 1 565 966
- PL-A- 157 185
- US-A- 4 128 658
- US-A- 4 521 431
- US-A- 5 338 871
- DATABASE WPI Week 8741 Derwent Publications Ltd., London, GB; AN 87-286326 & ES-A-8 706 656 (CHEMICA SA) , 16 September 1941

## Description

### Field of the Invention

This invention relates to a process for manufacturing Form 1 of the H₂ -antagonist 'Ranitidine hydrochloride' (N-[2-[[[5-(Dimethylamino)methyl]-2-furanyl]methyl]thio]ethyl-N'-methyl-2-nitro-1,1-ethenediamine) hydrochloride, at a commercial scale.

### Background of the Invention

Ranitidine hydrochloride, as described and claimed in British Patent Specification No. 1,565,966, (Apr. 1980) and U.S. Pat. No. 4,128,658 (Dec. 1978) shows potent histamine H₂-blocking activity.

A process for preparing ranitidine hydrochloride is known and described in U.S. Pat. No. 4,128,658 (Dec. 1978) and British Patent Specification No. 1,565,966 (Apr. 1980). In U.S. Patent No. 4,521,431 (June 1985), the ranitidine hydrochloride produced by the method described in U.S. Patent No. 4,128,658 (Dec. 1978) and British Patent Specification No. 1,565,966 (Apr. 1980) was designated as crystalline Form 1 of ranitidine hydrochloride.
A process for preparing crystalline Form 2 ranitidine hydrochloride was disclosed in U.S. Pat. No. 4,521,431 (June 1985), which is now commercially produced and marketed by Glaxo Holdings, the owner of the foregoing patents.

According to Glaxo, the described method to produce Form 1 ranitidine hydrochloride in U.S. Pat. No. 4,128,658 (Dec. 1978), does not have the desirable features of a manufacturing process and the product has unsuitable filtration and drying characteristics. During recent legal proceedings, Glaxo has also stated that it is not possible to manufacture crystalline Form 1 ranitidine hydrochloride on a commercial scale and moreover, that Form 1 is unstable and gets converted into stable Form 2 quickly. All attempts made so far by many researchers around the world to produce Form 1 ranitidine hydrochloride as per the described method in U.S. Pat. No. 4,128,658 (Dec. 1978) in the laboratory have failed.

EP-A-626 381 which is state of art according to Art. 54 (3) (4) EPC discloses a process for preparing Form 1 ranitidine hydrochloride which comprises forming a solution of ranitidine hydrochloride in a mixed solvent comprising at least one C₁-C₄ alkanol and a C₆-C₁₀ aromatic hydrocarbon and initiating crystallisation of Form 1 ranitidine hydrochloride from said solution in the presence of seed crystals of pure form 1 ranitidine hydrochloride. Chemical Abstract, vol. 119, 160082g discloses preparation of highly pure ranitidine whereby a solution of crude ranitidine in a water-immiscible, halogenated solvent, preferably chloroform, is treated under stirring with excess HCl and whereby the layers are separated and the crystalline ranitidine hydrochloride is precipitated from the aqueous layer by Me₂CHOH or a mixture of Me₂CO-MeOH. ES-A-8 706 656 discloses dissolving of ranitidine base in the amorphous state in absolute ethanol and reaction with HCl gas, free from water, at ambient temperature. The product is cooled to 4°C for crystallization separated and purified by conventional means.

WO 95/27709 which is state of the art according to Art. 54 (3), (4) EPC discloses the production of an improved form of Form 1 ranitidine hydrochloride wherein at first ranitidine free base is dissolved in a substantially anhydrous hydroxylic solvent and in a second process step anhydrous hydrogen chloride gas or a solution of anhydrous hydrogen chloride gas is added to the substantially anhydrous hydroxylic solvent containing ranitidine free base.

### SUMMARY OF THE INVENTION

The present invention provides a process for producing Form 1 ranitidine hydrochloride which is convenient to operate on a commercial scale and more economical than the method described in U.S. 4,128,658 (Dec. 1978).

The process according to the present invention comprises, dissolving ranitidine base at about 10°C to reflux temperature in a "suitable solvent" containing hydrogen chloride, stirring for some more time, and collecting the resulting product.

### BRIEF DESCRIPTION OF THE DRAWINGS

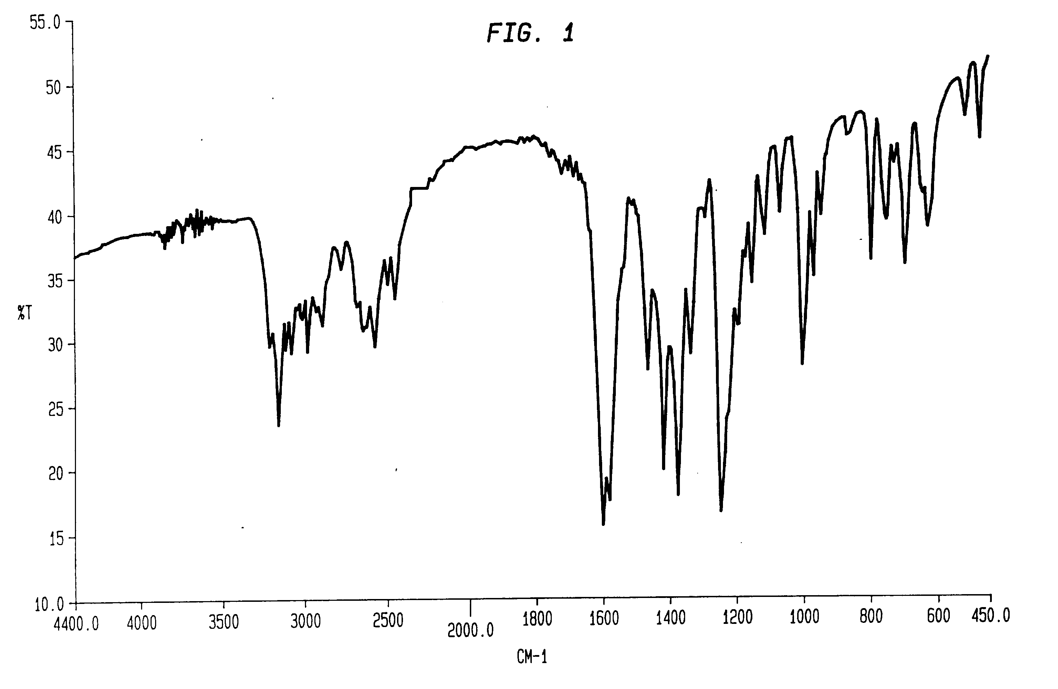

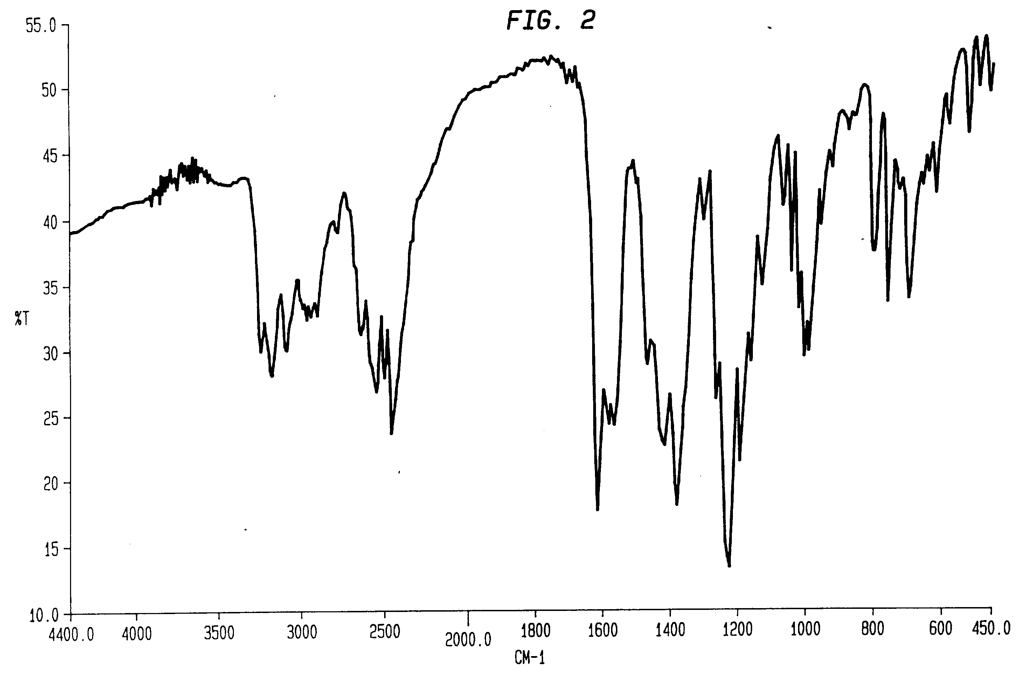

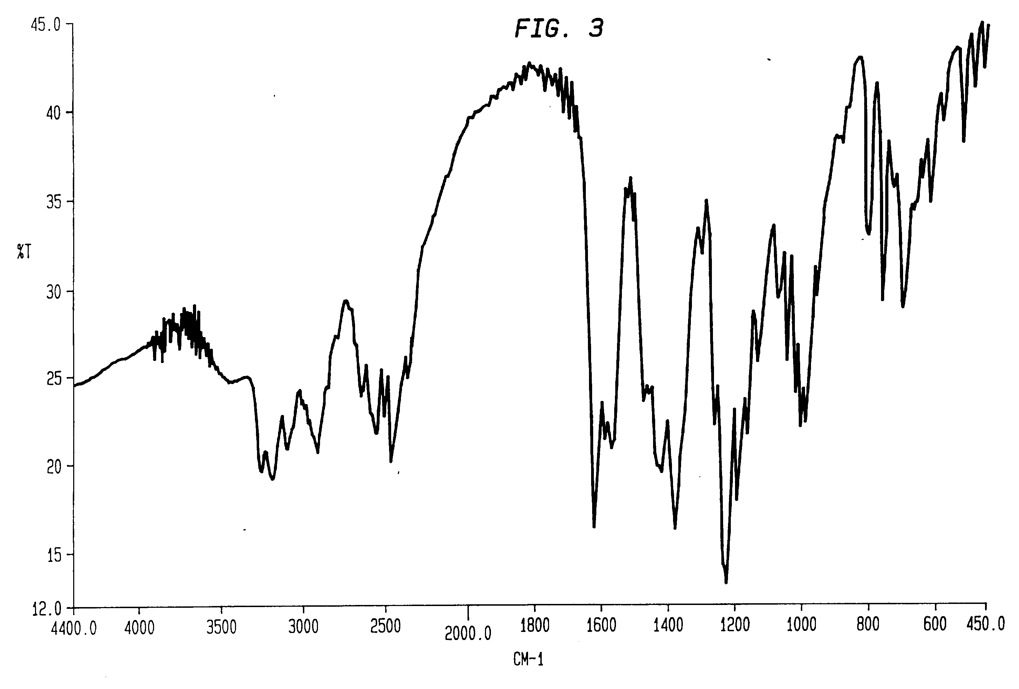

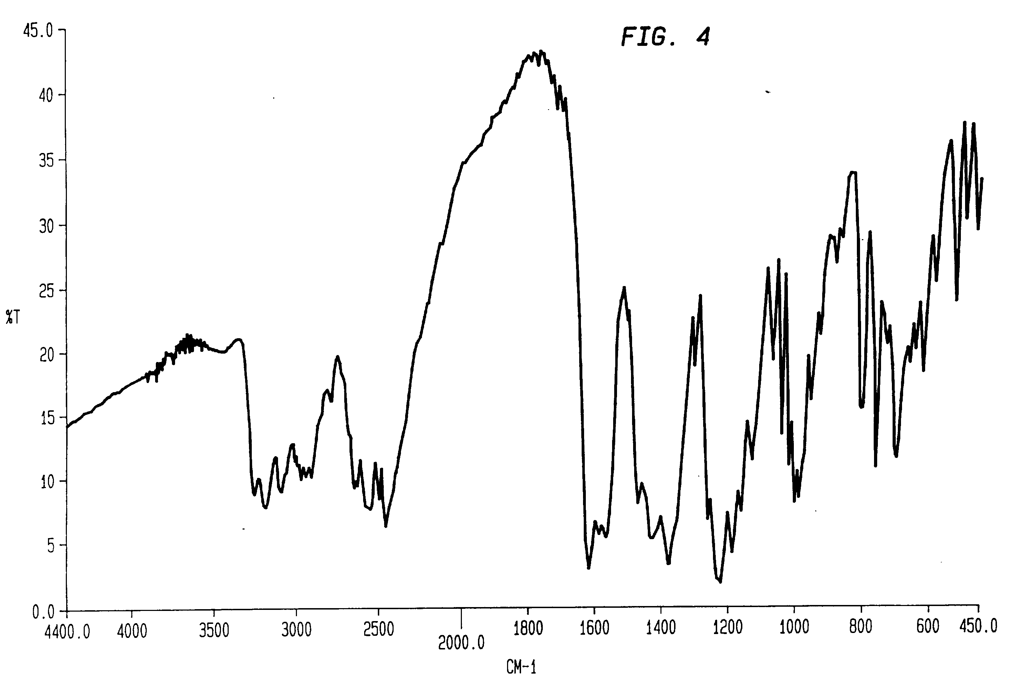

Form 1 ranitidine hydrochloride produced according to this method was characterized by its infra-red spectrum in KBr disc (Fig.1) and/or by its X-ray powder diffraction pattern (Table I below). Figs. 2, 3, and 4 show the infra-red spectra in KBr disc of Form 2 ranitidine hydrochloride viz USP reference standard sample CAT No. 59840, Lot F, Zantac® Tablets, Glaxo USA, B.No.Z12130MP and Ranbaxy India, B.No.:RTDT-01494, respectively. Fig. 5 shows comparative dissolution rates of 150mg Zantac® (Glaxo) tablets vs. 150mg tablets of Form 1 and Form 2 ranitidine hydrochloride, the Form 1 ranitidine hydrochloride being produced in accordance with the process of this invention.

### DETAILED DESCRIPTION OF THE INVENTION

In accordance with the present invention, Form 1 ranitidine hydrochloride is produced by dissolving ranitidine base at a temperature of about 10°C to reflux temperature in a "suitable solvent", such as a lower alkanol, containing hydrogen chloride, stirring, and collecting the resulting product.

It has been found that if the Form 1 ranitidine hydrochloride is prepared under the above defined working conditions, the following advantages are obtained:
(1) the product is easily filtrable and can readily be dried;
(2) the solvents used are readily recoverable;
(3) the process is economical and convenient to operate on a plant scale;
(4) the product has a high degree of purity (>99.5%); and
(5) the product is stable.

The term "suitable solvent" means any lower alkanol having from 3 to 5 carbon atoms and includes primary, secondary and tertiary alcohols. Suitable lower alkanol solvents include n-propanol, isopropanol, n-butanol, isobutanol, amyl alcohol and t-butanol. Preferably, the lower alkanol solvent used in the preparation of Form 1 ranitidine hydrochloride will be n-propanol, isopropanol or n-butanol. Isopropanol is the preferred solvent. Mixtures of two or more lower alkanols and/or other solvents such as hydrocarbon solvent, that is a solvent containing only the elements of carbon and hydrogen such as hexane, benzene, toluene and xylene, or a ketone or ester, having from one to ten carbon atoms such as acetone, methyl ethyl ketone, 2-butanone, 4-mthylpentan-2-one, ethyl acetate, n-butyl acetate or acetonitrile are also contemplated.

Generally, the reaction is carried out in a "suitable solvent" as a medium that has been heated by standard means to a temperature of from about 10°C to reflux temperature, preferably to about 35-70°C, most preferably to about 40-50°C. The amount of solvent is at least 1 part by volume per part of the starting material. Higher amounts of solvents and generally up to 20 parts by volume can be used. Amounts higher than 20 volumes are not useful from an economical point of view because large size apparatus would be necessary. In general, molar equivalent proportions of hydrogen chloride and ranitidine should be used but varying amounts of molar concentrations are within the scope of this invention.

The precipitation will typically be accomplished within about 5 minutes to about 5 hours. However, the length of time required will vary depending on such factors as total volume of solution, size of batch and container, temperature of the reaction, and presence or absence of stirring.

The present invention also provides a process for the conversion of Form 2 ranitidine hydrochloride to stable Form 1 using similar conditions for crystallization as those described above which give Form 1 ranitidine hydrochloride. Form 2 ranitidine hydrochloride may be dissolved in a lower alkanol solvent, selected from the group consisting of ethanol, n-propanol, isopropanol, n-butanol, isobutanol, t.-butanol and amyl alcohol, or a mixture of such solvents, by warming, followed by stirring and cooling until crystallization is complete. Addition of a miscible solvent such as ethyl acetate to the solution can be advantageously used to complete crystallization. Methods known in the art may be used with the process of this invention to enhance any aspect of this process. For example, the solution may be seeded with one or more crystals of Form 1 ranitidine hydrochloride prior to the initiation of product crystallization or the slurry may be cooled prior to filtration.

Generally, the product can be collected by any standard method known in the art such as by filtration, filtration under vacuum, or decantation and drying. Typically, this product will be collected by filtration when any of the solvents within the scope of this process are used.

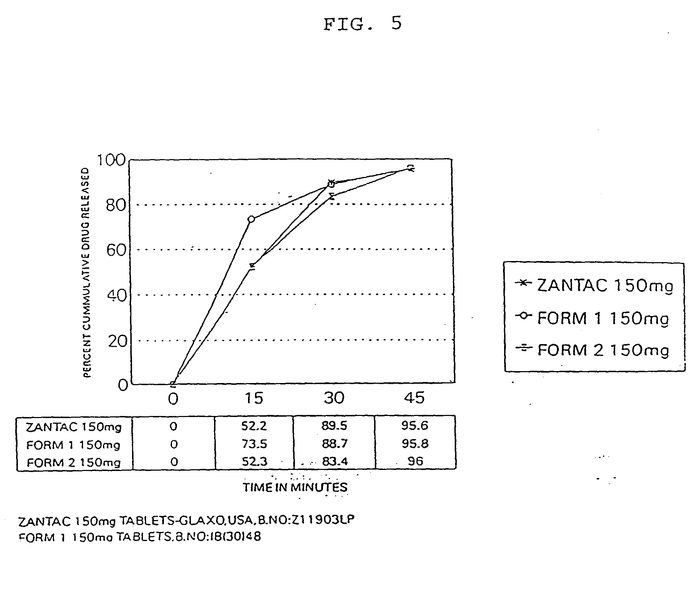

The tablets (150mg each) produced from two polymorphs, Form 1 and Form 2, of ranitidine hydrochloride, and Zantac® of Glaxo both exhibit equivalent dissolution and high solubility in water as shown in FIG. 5. Form 1 and Form 2 are known to have indistinguishable bioavailability (Ph.D. Thesis, "A Study of the Stability of Ranitidine," p.21, Dec. 1987, submitted by Philip Andrew Haywood to the Council for National Academic Awards, The Hatfield Polytechnic, Hatfield, Hertfordshire, U.K.).

The following specific examples are presented to illustrate the process.

### EXAMPLE 1

N-[2-[[[5-(Dimethylamino)methyl-2-furanyl]methyl]thio]ethyl]-N'-methyl-2-nitro-1,1-ethenediamine (100g) was dissolved in isopropanol (800ml) containing molar equivalent of hydrogen chloride at 40-50°C. The solution was stirred further for 1 hr. The hydrochloride crystallized during this period. The product was filtered off, washed with isopropanol twice (100ml each) and was dried at 40°C under vacuum to give Form 1 ranitidine hydrochloride (106g), m.p. 137-138°C, DSC peak temperature=143.4°C, purity=99.6% (HPLC).

### EXAMPLE 2

The process of Example 1 was repeated, using n-butanol instead of isopropanol to give Form 1 ranitidine hydrochloride (5.12g), m.p. 136-137°C, purity=99.5% (HPLC).

### EXAMPLE 3

N-[2-[[[5-(Dimethylamino)methyl-2-furanyl]methyl]thio]ethyl]-N'-methyl-2-nitro-1,1-ethenediamine (5g) was dissolved in n-propanol (40 ml) containing molar equivalent of hydrogen chloride. Ethyl acetate (20ml) was added slowly to the solution. It was further stirred for 1 hr. Form 1 ranitidine hydrochloride crystallized during this period, and was filtered off. The product was washed with ethyl acetate (20ml) and was dried at 40°C under vacuum to give Form 1 ranitidine hydrochloride (5g), m.p. 136-137°C, DSC peak temperature=144°C, purity=99.6% (HPLC).

### EXAMPLE 4

The process of Example 3 was repeated, using ethanol (20ml) instead of n-propanol to give Form 1 ranitidine hydrochloride (5.06g), m.p. 136-137°C, purity=99.8% (HPLC).

### EXAMPLE 5

N-[2-[[[5-(Dimethylamino)methyl-2-furanyl]methyl]thio]ethyl]-N'-methyl-2-nitro-1,1-ethenediamine (5g) was dissolved in isopropanol (40 ml) containing molar equivalent of hydrogen chloride. Toluene (20ml) was added slowly to the solution. It was further stirred for 1 hr. Form 1 ranitidine hydrochloride crystallized during this period. The product was filtered off, washed with toluene (20ml) and was dried at 45°C under vacuum to give Form 1 ranitidine hydrochloride (5.08g), m.p. 137-138°C, DSC peak temperature=143.1°C, purity=99.7% (HPLC).

### EXAMPLE 6

The process of Example 5 was repeated, using 4-methylpentan-2-one instead of toluene to give Form 1 ranitidine hydrochloride (5.17g), m.p. 137-138°C, DSC peak temperature =142.3°C, purity=99.6% (HPLC).

### EXAMPLE 7

N-[2-[[[5-(Dimethylamino)methyl-2-furanyl]methyl]thio]ethyl]-N'-methyl-2-nitro-1,1-ethenediamine (5g) was dissolved in n-propanol (20 ml). A further quantity of n-propanol (20ml) containing molar equivalent of hydrogen chloride was added slowly to the solution. The solution was seeded with Form 1 crystals at this temperature to induce crystallization. It was stirred further for 1-2 hrs. The hydrochloride crystallized during this period, and was filtered off. The product was washed with n-propanol (2x5ml) and was dried at 45°C under vacuum to give Form 1 ranitidine hydrochloride (4.49g), m.p. 136-137°C, purity=99.6% (HPLC).

### EXAMPLE 8

N-[2-[[[5-(Dimethylamino)methyl-2-furanyl]methyl]thio]ethyl]-N'-methyl-2-nitro-1,1-ethenediamine (1.0kg) was dissolved in isopropanol (8.0lt) containing molar equivalent of hydrogen chloride. The solution was stirred further for 1-2 hrs. The hydrochloride crystallized during this period, and was filtered off. The product was washed with isopropanol twice (1.0lt each) and was dried at 45°C under vacuum to give Form 1 ranitidine hydrochloride (1.05kg), m.p. 136-137°C, DSC peak temperature =143.3°C, purity=99.8% (HPLC).

### EXAMPLE 9

### CONVERSION OF RANITIDINE HYDROCHLORIDE FORM 2 TO STABLE FORM 1

Form 2 ranitidine hydrochloride (50g) was dissolved in ethanol (750ml) at 60-65°C. It was followed by the slow addition of ethyl acetate (750ml). The temperature came down to 45°C and the solution was seeded with Form 1 crystals at this temperature to induce crystallization. The solution was stirred for 1 hr. and then cooled to 15-20°C. The product was filtered off, washed with ethyl acetate (100ml) and dried at 40°C under vacuum to give stable Form 1 ranitidine hydrochloride (38.7g), m.p. 136-138°C, purity=99.6%(HPLC).

### EXAMPLE 10

The process of Example 9 was repeated at a 5 g scale, using ethanol (60ml, containing 5% v/v methanol) to give Form 1 ranitidine hydrochloride (4.36g), m.p. 137-138°C, purity=99.7% (HPLC).

### EXAMPLE 11

### CONVERSION OF RANITIDINE HYDROCHLORIDE FORM 1 TO FORM 2

Form 1 ranitidine hydrochloride (5g) was dissolved in methanol (7.5ml) at 50°C. Acetone (30ml) was added slowly to the solution at 50°C and the product was allowed to crystallize at 50°C. The slurry was cooled to 10-12°C and the product was filtered off, washed with acetone (2x5ml), and dried at 50°C under vacuum to give Form 2 ranitidine hydrochloride (4.5g), m.p. 139-140°C, purity=99.7% (HPLC).

**TABLE 1:**

| X-RAY DIFFRACTION DATA OF FORM 1, FORM 2 & USP REFERENCE STANDARD RANITIDINE HYDROCHLORIDE | | | | | | |
|---|---|---|---|---|---|---|
| INTERPLANAR DISTANCE d(Å) | | | | RELATIVE INTENSITY (I/I₀) | | |
| FORM 1 | FORM 2 | USP REF.STD | | FORM 1 | FORM 2 | USP REF.STD |
| 6.188 | 10.773 | 10.906 | | 29 | 9 | 13 |
| 5.862 | 6.188 | 6.188 | | 28 | 11 | 17 |
| 5.675 | 5.901 | 5.862 | | 41 | 18 | 26 |
| 5.273 | 5.433 | 5.4 | | 96 | 27 | 37 |
| 5.121 | 4.978 | 4.951 | | 35 | 13 | 15 |
| 4.287 | 4.716 | 4.741 | | 31 | 15 | 13 |
| 4.11 | 4.435 | 4.435 | | 98 | 100 | 100 |
| 3.983 | 4.287 | 4.287 | | 70 | 20 | 27 |
| 3.735 | 3.931 | 3.931 | | 26 | 19 | 23 |
| 3.615 | 3.814 | 3.814 | | 100 | 53 | 81 |
| 3.49 | 3.72 | 3.72 | | 46 | 25 | 41 |
| 3.411 | 3.63 | 3.615 | | 59 | 15 | 21 |
| 3.161 | 3.476 | 3.476 | | 60 | 23 | 33 |
| 3.086 | 3.36 | 3.36 | | 41 | 12 | 16 |
| 2.837 | 3.264 | 3.264 | | 42 | 25 | 32 |
| 2.047 | 3.14 | 3.14 | | 20 | 24 | 24 |
| | 3.055 | 3.055 | | | 12 | 14 |
| | 2.937 | 2.937 | | | 9 | 13 |
| | 2.891 | 2.891 | | | 12 | 18 |
| | 2.82 | 2.82 | | | 21 | 25 |
| | 2.728 | 2.72 | | | 12 | 16 |
| | 2.479 | 2.479 | | | 15 | 17 |
| | 2.446 | 2.44 | | | 12 | 16 |
| | 2.313 | 2.307 | | | 9 | 11 |

## Claims

1. A process for producing stable Form 1 ranitidine hydrochloride comprising dissolving ranitidine base in an organic solvent wherein hydrogen chloride is already dissolved and collecting crystalline Form 1 ranitidine hydrochloride from said solvent, wherein said solvent is a lower alkanol having 3 to 5 carbon atoms, a mixture of two or more lower alkanols or the lower alkanol with an additional organic solvent selected of a hydrocarbon solvent, a ketone or ester having from 1 to 10 carbon atoms.

2. The process of claim 1 wherein said lower alkanol is n-propanol, isopropanol, n-butanol, t-butanol, amyl alcohol, or mixtures thereof.

3. The process of claim 1 wherein said lower alkanol is isopropanol.

4. The process of claim 1 wherein said additional organic solvent is selected from the group consisting of a hydrocarbon solvent such as hexane, benzene, toluene, and xylene or a ketone or ester having from 1 to 10 carbon atoms such as acetone, methyl ethyl ketone, 2-butanone, 4-methyl pentan-2-one, ethyl acetate, n-butyl acetate or acetonitrile, and mixtures thereof.

5. The process of claim 1 wherein said ranitidine base is dissolved in said organic solvent at a temperature from about 10°C to reflux temperature.

6. The process of claim 1 wherein said Form 1 ranitidine hydrochloride is collected by filtration.

7. A process for producing stable Form 1 ranitidine hydrochloride comprising dissolving Form 2 ranitidine hydrochloride in an organic solvent and crystallizing Form 1 ranitidine hydrochloride from said organic solvent, wherein said organic solvent comprises at least one lower alkanol selected from the group consisting of ethanol, n-propanol, isopropanol, n-butanol, isobutanol, amylalcohol and t-butanol.

8. The process of claim 7 further comprising adding a miscible solvent to said solvent in which said Form 2 ranitidine hydrochloride has been dissolved.

9. The process of claim 8 wherein said miscible solvent is ethyl acetate.

10. The process of claim 7 further comprising warming said organic solvent to facilitate dissolving said Form 2 ranitidine hydrochloride therein.

11. The process of claim 7 further comprising adding said crystals of Form 1 ranitidine hydrochloride to said organic solvent in order to facilitate crystallization of said Form 1 ranitidine hydrochloride from said solvent.

12. The process of claim 11 further comprising cooling said solvent during said crystallization step.

## Patentansprüche

1. Verfahren zur Herstellung von stabilem Form 1 Ranitidinhydrochlorid, das ein Auflösen der Ranitidinbase in einem organischen Lösungsmittel, in welchem bereits Chlorwasserstoff gelöst ist, und ein Sammeln des kristallinen Form 1 Ranitidinhydrochlorids aus diesem Lösungsmittel beinhaltet, worin das Lösungsmittel ein niederer Alkanol mit 3 bis 5 Kohlenstoffatomen, eine Mischung aus zwei oder mehreren niederen Alkanolen oder des niederen Alkanols mit einem weiteren organischen Lösungsmittel ausgewählt aus einem Kohlenwasserstofflösungsmittel, einem Keton oder Ester mit 1 bis 10 Kohlenstoffatomen ist.

2. Verfahren nach Anspruch 1, worin der niedere Alkanol n-Propanol, Isopropanol, n-Butanol, tert.-Butanol, Amylalkohol oder eine Mischung hieraus ist.

3. Verfahren nach Anspruch 1, worin der niedere Alkanol Isopropanol ist.

4. Verfahren nach Anspruch 1, worin das zusätzliche organische Lösungsmittel ausgewählt ist aus der Gruppe bestehend aus einem Kohlenwasserstofflösungsmittel, wie Hexan, Benzol, Toluol und Xylol oder einem Keton oder Ester mit 1 bis 10 Kohlenstoffatomen, wie Aceton, Methylethylketon, 2-Butanon, 4-Methylpentan-2-on, Ethylacetat, n-Butylacetat oder Acetonitril und Mischungen hieraus.

5. Verfahren nach Anspruch 1, worin die Ranitidinbase in dem organischen Lösungsmittel bei einer Temperatur von etwa 10°C bis zur Rückflußsiedetemperatur erhitzt wird.

6. Verfahren nach Anspruch 1, worin das Form 1 Ranitidinhydrochlorid durch Filtrieren gesammelt wird.

7. Verfahren zur Herstellung von stabilem Form 1 Ranitidinhydrochlorid, das ein Auflösen von Form 2 Ranitidinhydrochlorid in einem organischen Lösungsmittel und ein Kristallisieren von Form 1 Ranitidinhydrochlorid aus diesem organischen Lösungsmittel beinhaltet, worin das organische Lösungsmittel mindestens einen niederen Alkanol enthält, der ausgewählt ist aus der Gruppe bestehend aus Ethanol, n-Propanol, Isopropanol, Isopropanol, n-Butanol, Isobutanol, Amylalkohol und tert.-Butanol.

8. Verfahren nach Anspruch 7, das weiterhin die Zugabe eines mischbaren Lösungsmittels, in dem das Form 2 Ranitidinhydrochlorid gelöst wurde, zu dem Lösungsmittel enthält.

9. Verfahren nach Anspruch 8, worin das mischbare Lösungsmittel Ethylacetat ist.

10. Verfahren nach Anspruch 7, das weiterhin ein Aufwärmen des organischen Lösungsmittels beinhaltet, um das Auflösen des Form 2 Ranitidinhydrochlorids darin zu vereinfachen.

11. Verfahren nach Anspruch 7, das weiterhin die Zugabe der Form 1 Ranitidinhydrochloridkristalle zu dem organischen Lösungsmittel beinhaltet, um die Kristallisation des Form 1 Ranitidinhydrochlorids aus dem Lösungsmittel zu erleichtern.

12. Verfahren nach Anspruch 11, das weiterhin ein Abkühlen des Lösungsmittels während des Kristallisationsschrittes beinhaltet.

## Revendications

1. Procédé de production de la forme 1 stable du chlorhydrate de ranitidine comprenant une étape de dissolution de la ranitidine base dans un solvant organique dans lequel de l'acide chlorhydrique est déjà dissous, et l'étape de récolte de la forme 1 du chlorhydrate de ranitidine à partir dudit solvant, dans lequel ledit solvant est un alcanol inférieur possédant de 3 à 5 atomes de carbone, un mélange de deux ou plus d'alcanols inférieurs ou l'alcanol inférieur avec un solvant organique supplémentaire choisi parmi les solvants hydrocarbure, une cétone ou un ester possédant de 1 à 10 atomes de carbone.

2. Procédé de production selon la revendication 1 dans lequel ledit alcanol inférieur est le n-propanol, l'isopropanol, le n-butanol, le t-butanol, l'alcool amylique ou des mélanges de ces derniers.

3. Procédé selon la revendication 1 dans lequel l'alcanol inférieur est l'isopropanol.

4. Procédé selon la revendication 1 dans lequel ledit solvant organique est choisi dans le groupe constitué par les hydrocarbures tels l'hexane, le benzène, le toluène et le xylène ou une cétone ou un ester ayant de 1 à 10 atomes de carbone tel l'acétone, la méthyl éthyl cétone, la 2-butanone, la 4-méthyl pentan-2-one, l'acétate d'éthyle, l'acétate de n-butyle ou l'acétonitrile, et des mélanges de ces derniers.

5. Procédé selon la revendication 1 dans lequel ladite ranitidine base est dissoute dans ledit solvant organique à une température située entre environ 10°C et la température de reflux.

6. Procédé selon la revendication 1 dans lequel ladite ranitidine de forme 1 est récoltée par filtration.

7. Procédé de production de la forme 1 stable du chlorhydrate de ranitidine comprenant l'étape de dissolution du chlorhydrate de ranitidine de forme 2 dans un solvant organique et cristallisation du chlorhydrate de ranitidine de forme 1 à partir dudit solvant organique dans lequel ledit solvant comprend au moins un alcanol choisi dans le groupe constitué par l'éthanol, le n-propanol, l'isopropanol, le n-butanol, l'isobutanol, l'alcool amylique et le t-butanol.

8. Procédé selon la revendication 7 comprenant de plus l'addition d'un solvant miscible audit solvant dans lequel le chlorhydrate de ranitidine de forme 2 a été dissous.

9. Procédé selon la revendication 8 dans lequel ledit solvant miscible est l'acétate d'éthyle.

10. Procédé de la revendication 7 comprenant en outre le chauffage dudit solvant organique pour faciliter la dissolution dudit chlorhydrate de ranitidine de forme 2.

11. Procédé selon la revendication 7 comprenant en outre l'addition desdits cristaux de chlorhydrate de ranitidine de forme 1 audit solvant organique de façon à faciliter la cristallisation dudit chlorhydrate de ranitidine de forme 1 à partir dudit solvant.

12. Procédé selon la revendication 11 comprenant en outre le refroidissement dudit solvant durant l'étape de cristallisation.
